# EUROPÄISCHE PATENTSCHRIFT

(11) **EP 1 870 026 B1**
(45) Veröffentlichungstag und Bekanntmachung des Hinweises auf die Patenterteilung: **30.09.2015**
(21) Anmeldenummer: 06012693.5
(22) Anmeldetag: 21.06.2006
(51) Int. Cl.: A61B 5/00, A61N 1/08, G06F 13/22, H04L 12/28

(54) **Diabetescare-System zur Detektion eines Analyten und Verfahren zur selktiven Datenübertragung**
Diabetescare system for analyte detection and method to selectively transmit prioritized data.
Système pour la mesure des analytes concernant le diabète et méthode pour transmettre les données dans un ordre selectif

(43) Veröffentlichungstag der Anmeldung: 26.12.2007
(73) Patentinhaber: Roche Diagnostics GmbH, 68305 Mannheim (DE); F.Hoffmann-La Roche AG, 4070 Basel (CH)
(72) Erfinder: Rasch-Menges, Juergen, Dr., 68723 Schwetzingen (DE); Jansen, Paul, 68163 Mannheim (DE); Haar, Hans-Peter, Dr., 69168 Wiesloch (DE); Haueter,Ulrich, 3506 Grosshöchstetten (CH); Poredda, Andreas, Dr., 68259 Mannheim (DE)
(74) Vertreter: Petirsch, Markus

(56) Entgegenhaltungen:
- EP-A2- 1 437 674
- US-A- 5 564 429
- US-A1- 2001 027 384
- US-A1- 2004 122 488
- US-A1- 2004 215 270
- US-A1- 2006 122 469

## Beschreibung

Die Erfindung betrifft ein Diabetescare-System mit einer mobilen Komponente und einer Basisstation, wobei die mobile Komponente am Körper tragbar oder in den Körper implantierbar ist und einen Speicher zum Speichern von Daten, sowie eine Kommunikationseinheit zur Übertragung von Daten an die Basisstation einschließt. Die Basisstation weist eine Kommunikationseinheit zum drahtlosen Empfangen von Daten von der mobilen Komponente und eine Speichereinheit zum Abspeichern von übertragenen Daten auf. Optional kann eine Ausgabeeinheit zur Anzeige von Daten, die von der mobilen Komponente übertragen worden sind, vorgesehen sein. Eine Datenübertragung zwischen der mobilen Komponente und der Basisstation findet innerhalb einer Zeitspanne statt, in der eine drahtlose Kommunikationsverbindung besteht. Die Erfindung betrifft auch ein Verfahren zur selektiven Datenübertragung innerhalb eines Diabetescare-Systems mit einer mobilen Komponente und einer Basisstation.

Diabetescare-Systeme dienen zur Detektion eines für die Behandlung des Diabetes Mellitus relevanten Analyten im menschlichen Körper und/oder zur Therapie, insbesondere durch Injektion von Medikamenten. Wichtige Beispiele sind Blutglucosemeter oder Insulinpumpen. Moderne Diabetescare-Systeme unterstützen ihre Benutzer, indem durchgeführte Aktionen protokolliert werden und somit für eine spätere Auswertung zur Verfügung stehen. Auf diese Weise entstehen im Laufe einer längeren Benutzungsdauer sogenannte "history-files".

Ebenso wie die Protokolldaten von ausgeführten Aktionen können bei Diabetescare-Systemen Analysedaten abgespeichert werden. Speziell bei am Körper tragbaren (invasiven oder nicht-invasiven) und bei im Körper implantierten Analysesystemen findet eine häufige, oft sogar praktisch kontinuierliche Messung und Bestimmung des Analyten statt, bei der eine große Menge an Mess- bzw. Analysedaten gewonnen werden. Die zwischengespeicherten Daten müssen dann von Zeit zu Zeit an eine Basisstation, beispielsweise einen PC, übertragen werden.

Zu diesem Zweck werden bei am Körper tragbaren Geräten häufig drahtgebundene Schnittstellen oder drahtlose Schnittstellen mit entsprechenden Schnittstellenprotokollen verwendet. Bei implantierten Geräten erfolgt die Datenübertragung naturgemäß immer drahtlos. Bei der "klassischen" Datenübertragung wird typischerweise das gesamte vorhandene Datenmaterial heruntergeladen. Dabei findet eine sequentielle Übertragung statt, wobei die Daten in der Reihenfolge ihrer Erzeugung (first in - first out) oder in der umgekehrten Reihenfolge Erzeugung (last in - first out) übertragen werden.

Bei diesem klassischen Vorgehen wird vorausgesetzt, dass die Datenverbindung wenigstens für die Dauer des Übertragens aller Daten besteht. Sie darf in der Regel nicht unterbrochen werden. Der Benutzer ist sich bewusst, dass die Datenübertragung stattfindet, da er die Übertragung aktiv initiiert hat und überwacht den Vorgang. Die verwendete Software zur Datenübertragung zeigt den Verlauf der Übertragung zudem meist an, beispielsweise als Graphik und/oder als Prozentzahl.

Dieses Übertragen der Daten wird von den Benutzern als recht unkomfortabel empfunden. Sie sehen sich an die Basisstation "angebunden", da die Übertragung nicht unterbrochen werden soll und sie sich während der Datenübertragung nicht von der Basisstation entfernen können.

Das Dokument US2004/0122488 offenbart ein Diabetescare-System mit einer Basisstation und einer mobilen Komponente, die am Körper tragbar oder in den Körper implantierbar sein kann. Sie weist einen Speicher zum Speichern von Daten und eine Kommunikationseinheit zur Übertragung von Daten auf. Die Basisstation hat eine Kommunikationseinheit zum Empfang von Daten von der mobilen Komponente, wobei eine Übertragung von Daten stattfindet, wenn eine Kommunikationsverbindung besteht. Um die Datenrate zu erhöhen, wird eine Kompression der Daten in der mobilen Komponente vor der Übertragung durchgeführt.

Um den Bedienerkomfort zu erhöhen, werden Systeme gefordert, bei denen die Kommunikation zwischen der mobile Komponente, die am oder im Körper tragbar ist, und der Basisstation "on-demand" gestaltet ist, ohne dass sie von dem Benutzer initiiert oder überwacht werden muss. Dabei findet eine drahtlose Kommunikation automatisch statt, wenn der Benutzer die mobile Komponente in die Nähe der Basisstation bringt. Die Geräte erkennen die Nähe der jeweiligen Partnerkomponente automatisch. Der Verbindungsaufbau ist für den Benutzer deshalb sehr komfortabel und anwenderfreundlich.

Nur wenn sich der Benutzer nahe genug an der Basisstation aufhält, kann eine Datenübertragung durchgeführt werden. Die Verbindungsdauer, die für eine derartige Kommunikation zwischen einer am Körper getragenen mobilen Komponente und der Basisstation zur Verfügung steht, ändert sich in Abhängigkeit der Verweildauer des Benutzers in der Nähe der Basisstation. Somit ist die zur Datenübertragung nutzbare Zeitdauer dem System unbekannt. Mit den im Stand der Technik bekannten Diabetescare-Systemen ist eine zuverlässige Datenübertragung bei variierender und unbekannter Verbindungsdauer nicht möglich.

Bei der Verwendung von Diabetescare-Systemen besteht darüber hinaus eine besondere Problematik:
- Auf der einen Seite übernehmen diese Systeme lebenswichtige medizinische Funktionen mit hoher Komplexität. Ein Eingriff in das Regelungssystem des menschlichen Körpers, wie es aufgrund der von solchen System gewonnenen Daten oder von den Systemen direkt vorgenommen werden kann, wird sonst typischerweise von medizinisch hochqualifiziertem Personal und unter dessen ständiger Beobachtung vorgenommen, zum Beispiel auf einer Intensivstation. Der Bediener der Diabetescare-Systeme, beispielsweise einer Insulinpumpe, ist jedoch der Patient, der in der Regel ein medizinischer Laie ist und die Auswirkungen eines Eingriffs in das System nicht vollständig übersehen kann.
- Auf der anderen Seite verfügen Diabetiker aufgrund ihrer Krankheit, gerade im fortgeschrittenen Stadium, nur noch über eine eingeschränkte Motorik und eine reduzierte Aufmerksamkeit. Dies kann zu Fehlfunktionen und Fehleinstellungen der Geräte oder dazu führen, dass abweichende Einstellungen, wie beispielsweise eine falsch geregelte Insulinzugabe, nicht bemerkt werden. Dies kann schwere gesundheitliche Schäden bei den Patienten hervorrufen. Auch aus diesem Grund ist es wichtig, die Aktionen des Patienten zu protokollieren und regelmäßig an eine Basisstation zu übertragen, die Auswerte- und Alarmierungsfunktionen übernehmen kann.

Aufgrund der geschilderten Probleme besteht ein erhebliches Risiko, dass bei einer drahtlosen Kommunikationsverbindung zwischen dem am Körper getragenen Gerät und der Basisstation die gesamte Verbindungsdauer zum vollständigen Übertragen aller Daten nicht zuverlässig gewährleistet werden kann.

Es ist deshalb Aufgabe der vorliegenden Erfindung, ein Diabetescare-System vorzuschlagen, bei dem eine Datenübertragung von einer am Körper getragenen mobilen Komponente an eine Basisstation unter Berücksichtigung der erläuterten Probleme und Anforderungen mit erhöhtem Benutzerkomfort erreicht wird.

Gelöst wird die vorliegende Aufgabe durch ein Diabetescare-System mit den Merkmalen des Anspruchs 1 sowie durch ein Verfahren zur selektiven Datenübertragung mit den Merkmalen des Anspruchs 13.

Das erfindungsgemäße Diabetescare-System zur Anwendung am menschlichen Körper umfasst eine mobile Komponente und eine Basisstation. Die mobile Komponente ist am Körper tragbar oder in den Körper implementierbar und schließt einen Speicher zum Speichern von Daten und eine Kommunikationseinheit zur Übertragung von Daten an die Basisstation ein. Die Basisstation hat eine Kommunikationseinheit zum drahtlosen Empfangen von Daten von der mobilen Komponente und eine Speichereinheit zum Abspeichern von übertragenen Daten. Optional weist die Basisstation auch eine Ausgabeeinheit zur Anzeige von Daten auf, die von der mobilen Komponente übertragen worden sind. Die Datenübertragung zwischen der mobilen Komponente und der Basisstation erfolgt innerhalb einer Zeitspanne, in der eine drahtlose Kommunikationsverbindung besteht. Da die Zeitspanne zur Datenübertragung prinzipiell unbekannt ist, wird eine selektive Datenübertragung durchgeführt, wobei innerhalb der ersten Zeitspanne eine erste Teilmenge von Daten übertragen wird. Die erste Teilmenge der Daten wird mittels eines prozessorgesteuerten Selektionsalgorithmus derart selektiert, dass die in der Zeitspanne übertragenen Daten für die Gesamtheit der in der mobilen Komponente abgespeicherten Daten repräsentativ ist.

Im Rahmen der Erfindung wurde festgestellt, dass gerade bei Diabetes-care-Systemen mit einer kurzen Übertragungszeit zu rechnen ist, da der Diabetiker aufgrund eingeschränkter Aufmerksamkeit die drahtlose Kommunikation zwischen der am Körper getragenen mobilen Komponente und der Basisstation häufig nicht wahrnimmt bzw. überwacht. Deshalb ist die Datenmenge, die während der bestehenden Verbindung übertragen werden kann, häufig nur ein Bruchteil der in der mobilen Komponente gespeicherten Daten.

Durch geeignete Selektion der Daten kann schon mit einer relativ geringen Anzahl an selektierten und übertragenen Daten eine zuverlässige Aussage über die Gesamtheit der Daten gemacht werden. Dazu findet bevorzugt eine Priorisierung der Daten dergestalt statt, dass die Daten, deren Informationsgehalt am höchsten ist, bevorzugt übertragen werden. Dadurch, dass die in der ersten Teilmenge selektierten Daten die Gesamtheit der Daten abbilden, ist es nicht notwendig, alle Daten zu übertragen bzw. auszuwerten, um einen hinreichend genauen Informationsüberblick über die Gesamtheit der Daten zu erhalten.

Ein derartiges Diabetescare-System ist insbesondere dann vorteilhaft, wenn die mobile Komponente eine kontinuierlich arbeitende und messende Messeinheit aufweist, die im Körper implantiert ist. In dem Speicher der mobilen Komponente sind in der Regel Messwerte aus einem längeren Zeitraum, bis zu mehreren Tagen, enthalten. Aus dieser großen Zahl der Messwerte wird mit der vorliegenden Erfindung ein möglichst genaues Bild der Gesamtheit der Messwerte schon durch eine kleine Teilmenge erzeugt.

Da die Messwerte bzw. deren Auswertung zur Analyse und Bewertung von Regelgrößen dienen, nämlich beispielsweise bei Blutzuckermessgeräten zur Festlegung der zu verabreichenden Insulindosis, sind die aktuellsten Messwerte in der Regel besonders wichtig. Bevorzugt wird deshalb mindestens ein Teil der ersten Teilmenge der selektierten Daten oder Messwerte in einer Reihenfolge an die Basisstation übertragen, die umgekehrt zu der Reihenfolge ihrer Erzeugung ist ("last in - first out - Prinzip"). Der zuletzt gemessene Messwert wird zuerst übertragen. Dieses Prinzip muss nicht strikt angewendet werden, da es mit anderen Kriterien der Übertragung, die im Vordergrund stehen, kombiniert werden kann. Prinzipiell gilt auch hier, dass der Informationsgehalt eines Messwertes entscheidend ist, nicht allein der Zeitpunkt seiner Erstellung.

Um ein möglichst gutes Abbild der Gesamtheit der Daten zu erhalten, sind generell zwei Strategien möglich:
1. Die Auswahl bzw. Selektion der Daten aus der Gesamtheit kann mit einem festen Raster von Daten, die sich auf äquidistante Zeitpunkte beziehen, vorgenommen werden.
2. Die Daten der ersten Teilmenge werden derart selektiert, dass ein festes Raster vermieden wird, also der zeitliche Abstand zwischen zwei selektierten Daten nicht aquidistant ist.

In einer bevorzugten Ausführungsform, in der die erste Teilmenge der Daten in einem festen zeitlichen Raster selektiert wird, wobei der zeitliche Abstand zwischen zwei selektierten Daten äquidistant ist, wird eine gleichmäßige Übersicht der Daten aus der Gesamtheit erzeugt. Die Selektion der Daten kann dann, nachdem die erste Teilmenge der Daten von der mobilen Komponente an die Basisstation übertragen worden ist, sukzessive verfeinert werden, indem das Raster verschoben wird, beispielsweise so, dass der erste auszuwählende Wert zwischen zwei Daten der ersten Teilmenge liegt. Alternativ kann auch der äquidistante zeitliche Abstand zwischen den selektierten Daten verringert werden.

Die Selektion mit einem konstanten Raster bietet einen guten und schnellen Überblick über die Gesamtheit der Daten. Diese Methode ist insbesondere dann anzuwenden, wenn keiner der ausgewählten Messwerte auffällig erscheint. Allerdings werden bei einem konstanten Raster periodische Fehler nicht zuverlässig erkannt. Auch Ausreißer, also Daten, die oberhalb einer medizinisch akzeptablen Grenze liegen, oder Messfehler, werden bei einem festen Raster nicht unbedingt detektiert.

Vorteilhafterweise werden die Daten der ersten Teilmenge mittels eines Selektionsalgorithmusses selektiert, in dem eine Wahrscheinlichkeitsfunktion implementiert ist. Dabei ist der zeitliche Abstand zwischen je zwei zeitlich benachbarten selektierten Daten der ersten Teilmenge verschieden. Die selektierten Datgen der ersten Teilmenge werden gemäß einer Wahrscheinlichkeitsdichte ausgewählt.

In der Funktion können auch übergeordnete "Meta-Informationen" implementiert sein. Diese "Meta-Informationen" können von dem Diabetescare-System oder dem Analysesystem bzw. der mobilen Komponente selbst generiert werden, wenn es sich um ein "selbstlernendes" System handelt. Dazu werden aus der Vergangenheit und der Analyse der schon bei früheren Datenübertragungen ausgewählten und übertragenen Daten, beispielsweise Informationen darüber gewonnen, wann verstärkt Auffälligkeiten in Messwerten auftreten. Ist bei einem Patienten das Auftreten von auffälligen Werten am Morgen bisher verstärkt vorgekommen, so ist die Wahrscheinlichkeit dafür, dass auch bei den aktuell im Speicher der mobilen Komponente vorhandenen Werten auffällige Werte am Morgen detektiert wurden, erhöht. Der Selektionsalgorithmus kann derart verändert werden, dass insbesondere morgens verstärkt Werte abgefragt werden.

Dem Selektionsalgorithmus kann anstatt der Wahrscheinlichkeitsfunktion auch eine Zufallsfunktion zugrunde liegen, so dass die selektierten Daten der ersten Teilmenge "zufällig" ausgewählt werden. Der zeitliche Abstand je zweier benachbarter selektierter Daten ist dann in der Regel ebenfalls verschieden und nicht äquidistant, so dass die Probleme beim Auftreten periodischer Fehler vermieden werden.

Vorzugsweise wird aus bereits durchgeführten Datenübertragungen zwischen der mobilen Komponente und der Basisstation auch die voraussichtliche Dauer der ersten Zeitspanne für die Datenübertragung ermittelt. Ebenso kann aus bereits stattgefundenen Datenübertragungen die voraussichtliche Datenmenge, die in der Zeitspanne der bestehenden Kommunikationsverbindung übertragen werden kann, ermittelt werden. Diese Zeitdauer bzw. die Menge der Daten ist ein Maß für die Wahrscheinlichkeit dafür, dass auch bei der nun stattfindenden bzw. bei der nächsten Datenübertragung zwischen der mobilen Komponente und der Basisstation die gleiche Verbindungsdauer zur Verfügung steht bzw. die gleiche Anzahl an Daten übertragen werden können. Dabei wird davon ausgegangen, dass der Patient ein in etwa gleichbleibendes Verhaltensmuster zeigt. Insbesondere wenn mehrere bereits stattgefundene Datenübertragungen ausgewertet werden können, lässt sich die Vorhersage der nächsten Übertragungsdauer präzisieren. Somit lässt sich eine effektive Nutzung des zur Verfügung stehenden Zeitraums der Datenübertragung erreichen.

Bevorzugt wird die erste Teilmenge der Daten, die von der mobilen Komponente an die Basisstation während einer Datenübertragung übertragen worden ist, in dem Diabetescare-System registriert. Besonders vorteilhaft ist es, wenn sowohl in der mobilen Komponente als auch in der Basisstation die bereits übertragenen Daten vermerkt werden. Alternativ wird nur in einem der beiden Geräte ein Merker gesetzt. Wird eine Datenübertragung vorzeitig abgebrochen, beispielsweise weil sich der Benutzer mit seiner mobilen Komponente von der Basisstation entfernt, und dies auch nicht seinem vorhergesagten Verhaltensmuster entspricht, so kann bei der nächsten Datenübertragung die abgebrochene Übertragung fortgesetzt werden. In diesem Fall müssen bereits übertragene Daten nicht noch einmal von der mobilen Komponente an die Basisstation übermittelt werden. Die Effizienz der Datenübertragung von der mobilen Komponente zur Basisstation steigt dadurch. In einer bevorzugten Ausführungsform des erfindungsgemäßen Diabetescare-Systems werden die bereits übertragenen Daten der ersten Teilmenge aus dem Speicher der mobilen Komponente gelöscht.

Unter mobilen Komponenten, die "am Körper tragbar" sind, werden auch solche verstanden, die mit dem Körper in Kontakt gebracht werden. Ein Beispiel hierfür ist ein Injektionspen zum Spritzen eines Medikaments, beispielsweise Insulin, bei dem Dosierinformationen an eine Basisstation übertragen werden sollen. Ein derartiges mobiles Gerät wird nur kurzzeitig am Körper getragen, nämlich während der Applikation des Medikamentes.

Der Begriff "Daten" wird im Rahmen dieser Erfindung in seiner allgemeinen Form verstanden und umfasst alle Informationen, die zwischen einer mobilen Komponente und einer Basisstation eines Diabetiscare-Systems übertragen werden. Hierzu gehören beispielsweise Messwerte von Blutparametern, wie Blutzucker, Dosiervolumina, insbesondere von Injektionspens, Fördervolumina von Pumpen, insbesondere Insulinpumpen oder gemessene Druckverläufe. Ebenso sind auch Zeitwerte von dem Begriff "Daten" umfasst, die beispielsweise von einer internen Uhr oder einem Zähler generiert werden und als absolute oder relative Zeit vorliegen können. Auf diese Weise kann der Zeitpunkt der Erhebung der Messwerte erfasst und übertragen werden. Daten können darüberhinaus gemessene elektrische Widerstände, Spannungen oder Ströme sein. Auch optisch erfasste Werte (Photometrie) oder andere Zustandsinformationen sind im Rahmen dieser Erfindung unter den Begriff "Daten" subsumiert.

Eine bevorzugte Ausführungsform des erfindungsgemäßen Diabetescare-Systems wird anhand der nachstehenden Zeichnungen im Detail erläutert. Die dargestellten Besonderheiten können einzeln oder in Kombination verwendet werden, um bevorzugte Ausgestaltungen der Erfindung zu schaffen. Ohne Einschränkung der Allgemeinheit wird hier ein Analysesystem als eine mögliche Ausführungsform des Diabetescare-Systems beschrieben. Es zeigen:
- Fig. 1: ein Analysesystem mit einem Analysegerät und einer Basisstation;
- Fig. 2: eine Grafik des zeitlichen Verlaufs der in einem Speicher des Analysegerätes hinterlegten Messwerte;
- Fig. 3: einen zeitlichen Verlauf der Messwerte mit einem ersten Auswahlkriterium;
- Fig. 4: einen zeitlichen Verlauf der Messwerte mit einem zweiten Auswahlkriterium;
- Fig. 5: den zeitlichen Verlauf mit einem dritten Auswahlkriterium;
- Fig. 6: den zeitlichen Verlauf mit einem vierten Auswahlkriterium;

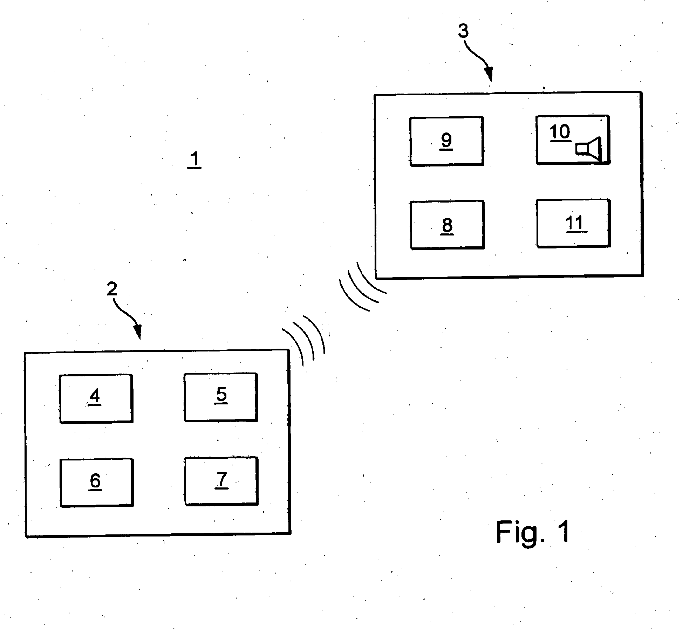

Das in Figur 1 dargestellte Analysesystem 1 umfasst als mobile Komponente ein am Körper tragbares oder implantierbares Analysegerät 2 und eine separate Basisstation 3.

Das Analysengerät 2 enthält eine Messeinheit 4 zur Detektion eines Analyten und zum Erzeugen von für die Analyse geeigneten Messwerte. Es schließt weiter eine Kommunikationseinheit 5, einen Prozessor 6 und einen Speicher 7 zur Speicherung von Messwerten ein. Die Messwerte bilden Daten, die bei bestehender Kommunikationsverbindung an die Basisstation übertragen werden. Eine erste Teilmenge der Messwerte wird mittels eines Selektionsalgorithmus selektiert, der von dem Prozessor 6 des Analysegerätes 2 gesteuert wird,

Bevorzugt wird die erste Teilmenge der Messwerte schon vor Beginn der Datenübertragung zwischen dem Analysegerät 2 und der Basisstation 3 selektiert. Damit stehen beim Aufbau einer Datenverbindung zwischen dem Analysegerät 2 und der Basisstation 3 alle zu übertragenen Messwerte zur Verfügung, so dass sie sofort übermittelt werden können.

Die Basisstation 3 umfasst eine Kommunikationseinheit 8 zum drahtlosen Empfangen von Messwerten des Analysegerätes 2, eine Speichereinheit 9 zum Abspeichern der von dem Analysegetät 2 übertragenen Messwerte und eine Ausgabeeinheit 10, mit der die Masswerte angezeigt werden. Die Ausgabeeinheit 2 kann neben einem Display auch einen Lautsprecher umfassen, um akustische Signale bzw. den Messwert in Form von Sprache auszugeben.

Vorzugsweise schließt die Basisstation 3 eine Meldeeinrichtung 11 ein, um eine optische und/oder akustische Warnmeldung auszugeben, wenn die Anzahl der übertragenen Messwerte eine untere Grenze unterschreitet. Die Meldeeinrichtung 11 kann ein Display, eine optische Lichtanzeige und/oder einen Lautsprecher umfassen. Die Wammeldung kann insbesondere erzeugt werden, wenn zu wenig Messwerte vom Analysegerät 2 zur Basisstation 3 übertragen worden sind, um eine zuverlässige Aussage über die Gesamtheit der in dem Analysengerät gespeicherten Messwerte zu treffen.

Zum Aufbau einer Kommunikationsverbindung zwischen der Kommunikationseinheit 5 des Analysegerätes 2 und der Kommunikationseinheit 8 der Basisstation 3, sendet die Basisstation 3 auf einer definierten Frequenz und mit einer definierten Sendeleistung eine Anfrage aus. Die Basisstation 3 wird dabei als "Master" bezeichnet, während das Analysegerät 2 der "Slave" ist, so dass eine "Master-Slave-Beziehung" zwischen den beiden Geräten besteht. Das Analysegerät 2 als Slave lauscht auf der gleichen Frequenz auf eine ankommende Anfrage von der Basisstation 3. Die Sendeleistung kann derart ausgelegt sein, dass das Analysegerät anhand der Zunahme der Signalstärke die Unterschreitung eines definierten Abstandes erkennt. Möglich ist auch eine Messung der bidirektionalen Datenübertragungszeit zwischen der Kommunikationseinheit 5 und der Kommunikationseinheit 8.

Wird der vorgegebene Abstand zwischen dem Analysegerät 2 und der Basisstation 3 unterschritten, so antwortet das Analysegerät 2 auf die Anfrage der Basisstation 3. Die Verbindung zwischen den Kommunikationseinheiten 5,8 wird aufgebaut. Optional kann eine Wartezeit vor Beginn der eigentlichen Datenübertragung festgelegt werden, z.B. um eine zu kurze oder eventuell ungewollte Verbindung zu erkennen oder um die Datenübertragungsparameter zwischen den beiden Geräten zu justieren, also die Geschwindigkeit der Datenübertragung, die Sendeleistung, etc. einzustellen.

Sind alle Voraussetzungen erfüllt, so sendet das Analysegerät 2 (vorzugsweise auch ohne eine direkte Anfrage von der Basisstation 3) die erste Teilmenge der Messwerte an den Master, also die Basisstation 3. Der Verzicht auf die Anfrage der Basisstation 3 verkürzt die zum Datenaustausch benötigte Zeit.

Vorzugsweise wird das Analysegerät 2 durch einen Impuls, ein Signal oder einem Befehl von der Basisstation 3 aus seinem "Ruhezustand" in einen "aktiven Zustand" versetzt. Der Ruhezustand ist ein Energiesparmodus oder ein Modus mit verringerter Sendeleistung. Dadurch wird zum einen die Strahlenbelastung des Patienten, der das Analysegerät 2 trägt, reduziert; zum anderen wird Energie gespart.

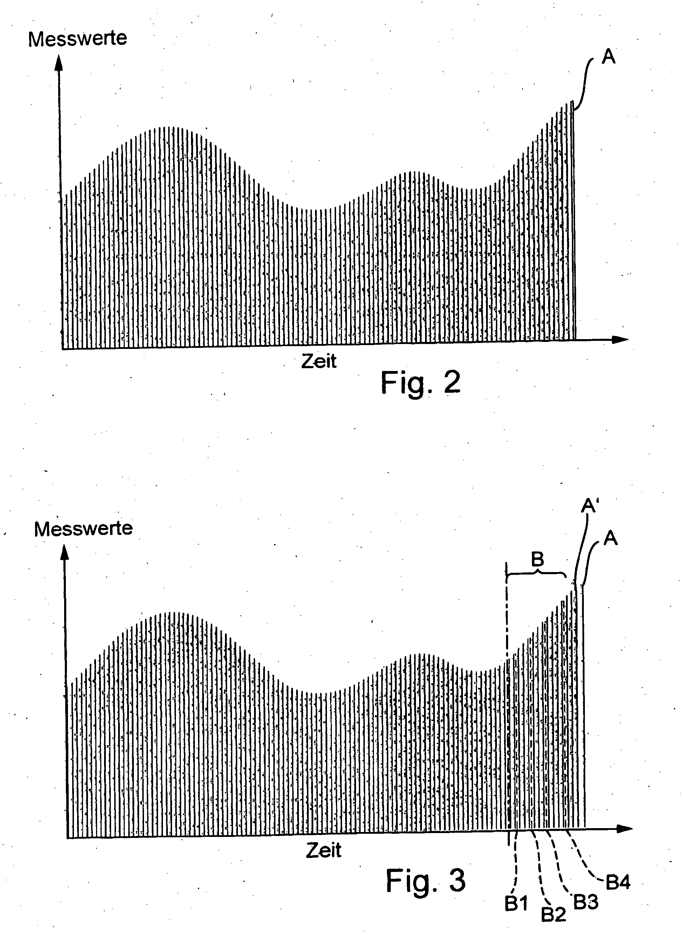

Figur 2 zeigt in dem Analysegerät 2 gespeicherte Messwerte. Dabei ist die Gesamtheit aller diskreten Messwerte dargestellt, die in äquidistante Zeitabschnitten gemessen worden sind.

Da bevorzugt die zu übertragenen Messwerte als erste Teilmenge bereits vor Aufbau der Datenübertragung selektiert sind, kann beim Bestehen einer Kommunikationsverbindung sofort mit der Datenübertragung zwischen dem Analysegerät 2 und der Basisstation 3 begonnen werden. Während der Datenübertragung arbeitet vorzugsweise das Analysegerät 2 als Master und die Basisstation als Slave. Beispielsweise kann der jüngste gemessene Messwert A aus der ersten Teilmenge zuerst übertragen werden. Möglich ist auch, einen anderen Wert aus der ersten Teilmenge als ersten Wert zu übertragen, beispielsweise einen als auffällig detektierten Messwert. Liegt kein Kriterium vor, aufgrund dessen ein Messwert wegen seines erhöhten Informationsgehaltes zuerst übermittelt wird, so wird der jüngste gemessene Messwert verwendet.

Nach der Übertragung des ersten Messwertes kann die Basisstation 3 optional ein Signal ausgeben, um dem Patienten mitzuteilen, dass ein Wert übertragen wurde. Bevorzugt kann der Patient selbst einstellen, ob eine Meldung der Basisstation 3 erfolgen soll. Falls eine derartige Rückmeldung gewünscht ist, kann sie entweder nach der Übertragung des ersten und/oder jedes weiteren Messwertes oder am Ende der durchgeführten Datenübertragung erfolgen.

Bricht die Kommunikationsverbindung nach dem Übertragen des ersten bzw. während der Übertragung der weiteren Werte ab, so wird das während der Datenübertragung als Master arbeitende Analysegerät 2 wieder zum Slave und fällt nach einer optionale Wartezeit wieder in seinen Ruhezustand zurück. Die Basisstation 3 wird wieder zum Master, so dass das Analysegerät 2 und die Basisstation 3 stets zwischen der Funktion des Masters und des Slaves hin- und herschalten.

Wurde die erste Teilmenge der selektierten Messwerte vollständig übertragen, so kann ein Bestätigungssignal zwischen dem Analysegerät 2 und der Basisstation 3 ausgetauscht werden. Die Basisstation 3 kann daraufhin weitere Messwerte von dem Analysegerät 2 anfordern. Alternativ kann das Analysegerät 2 auch so lange weitere Messwerte übertragen, bis die Gesamtheit der in ihrem Speicher befindlichen Messwerte an die Basisstation 3 übermittelt worden ist.

Bei der Selektion der ersten Teilmenge der Messwerte ist zu beachten, dass die Gesamtdauer der Kommunikationsverbindung stets unbekannt ist und mit einem vorzeitigen Abbruch jederzeit gerechnet werden muss. Die Selektion richtet sich deshalb vorzugsweise nach für eine medizinische Beurteilung notwendigen Anforderungen. Aus diesem Grund sind häufig die unmittelbar vor der Datenübertragung gemessenen Werte von Interesse. Bei der Ermittlung eines auffälligen Wertes, der über einem medizinisch festgelegten Grenzwert oder außerhalb eines Toleranzbandes liegt, wird dieser Wert bevorzugt übertragen. Das Toleranzband ist in dem System vorgegeben und kann vorzugsweise verändert werden, beispielsweise durch medizinisches Personal. Eine Veränderung durch die Patienten selbst ist in der Regel nicht gewünscht, aber möglich.

Die Selektion weiterer Messwerte wird in Abhängigkeit von einem bereits ausgewählten (selektierten) Messwert getroffen. Vorteilhaft ist diese abhängige Auswahl insbesondere, wenn der ausgewählte Wert als auffällig charakterisiert wird. Bevorzugt werden innerhalb eines Intervalls um einen auffälligen Messwert, der außerhalb eines vorgegebenen Toleranzbandes liegt, die weiteren Messwerte mit einem festen zeitlichen Raster selektiert. Besonders bevorzugt werden alle Messwerte innerhalb eines Intervalls um den auffälligen Messwert ausgewählt.

In Figur 3 werden die Messwerte A und A' als auffällige Werte erachtet, da sie oberhalb einer medizinisch indizierten Grenze liegen. Daraufhin wird das Intervall B ausgewählt, in dem die gestrichelt dargestellten Messwerte B1 bis B4 selektiert werden. Die äquidistante Auswahl der Messwerte im Intervall B vermittelt einen Überblick über den Werteverlauf in diesem Zeitraum und ermöglicht einen Anstieg oder einen Abfall zu dem auffälligen Wert A, A' hin zu erkennen. Auch hier könnte die Datenübertragung mit dem jüngsten Wert beginnen und so lange fortgesetzt werden, wie die Kommunikationsverbindung Bestand hat.

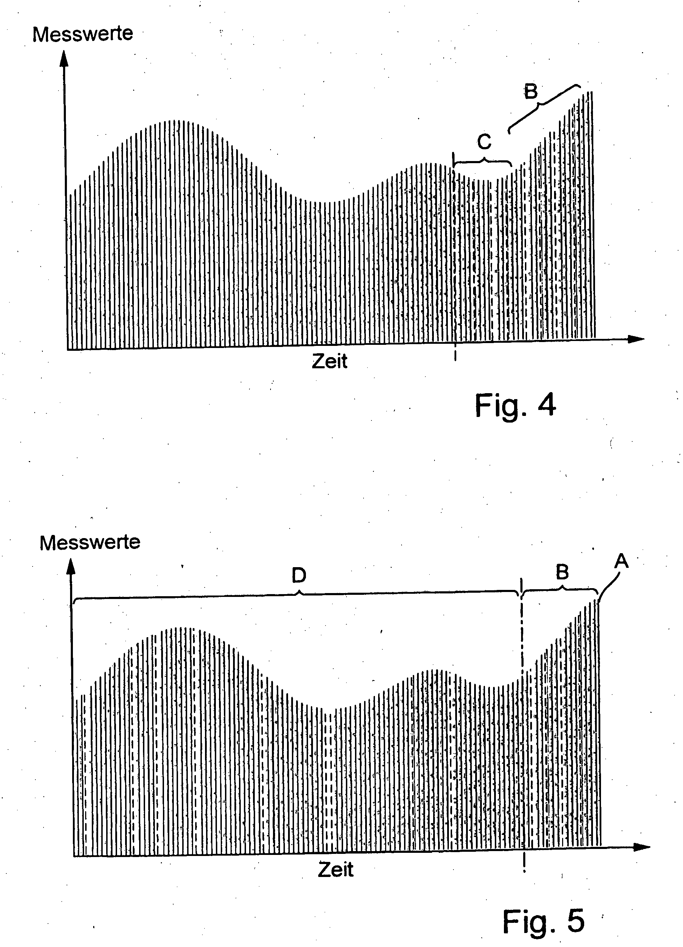

Um einen erweiterten Überblick zu erhalten, wird im Anschluß an das Intervall B ein weiteres Intervall C festgelegt, in dem ebenfalls Werte mit äquidistantem Raster selektiert werden. Hierdurch wird der Überblick über die Gesamtheit der Werte erweitert. Ein derartiges Vorgehen ist in Figur 4 dargestellt.

Vorteilhafterweise wird die Gesamtheit der in dem Speicher gespeicherten Messwerte in mehrere Intervalle unterteilt, wobei die erste Teilmenge aus Messwerten der Intervalle zusammengesetzt wird. Dabei werden die Messwerte derartig mit einer unterschiedlichen Dichte aus den Intervallen selektiert, dass eine Gewichtung der Intervalle erreicht wird. Die Auswahl von Messwerten mit einer unterschiedlichen Gewichtung aus verschiedenen Intervallen ist insbesondere dann vorteilhaft, wenn aus der Historie bekannt ist, dass in bestimmten Zeitintervallen häufiger auffällige Messwerte auftreten können. Aus diesen Intervallen wird dann eine relativ höhere Dichte an Messwerten selektiert, als aus anderen Intervallen, die sich in der Vergangenheit als eher unkritisch erwiesen haben.

Beispielsweise kann die Gesamtheit der gespeicherten Messwerte in zwei Intervalle unterteilt werden, wobei die Messwerte des ersten Intervalls jünger sind als die Messwerte des zweiten Intervalls. Die erste Teilmenge wird derart selektiert, dass die Dichte der aus dem ersten Intervall selektierten Messwerte größer ist als die Dichte der aus dem zweiten Intervall selektierten Messwerte. Hierdurch wird berücksichtigt, dass in der Regel die jüngeren Messwerte einen höheren Informations-gehalt enthalten. Insbesondere wenn die Messwerte dazu dienen, die Insulinabgabe zu dosieren, sind die jüngeren Messwerte von größerem Interesse als weiter zurückliegende Messwerte.

Die Aufteilung der Gesamtheit der gespeicherten Messwerte in zwei Intervalle ist in Figur 5 dargestellt. Das Intervall B umfasst eine erste Anzahl jüngerer Messwerte. Die im Intervall B zusammengefassten Messwerte spiegeln im dargestellten Beispiel etwa 15 Prozent der Gesamtheit der Messwerte wider. Aus diesen 15 Prozent werden die vier Messwerte B1 bis B4 selektiert. Das Intervall D umfasst die verbleibenden 85 Prozent der Messwerte. Aus diesem Intervall werden insgesamt 9 gestrichelt dargestellte Messwerte selektiert. Die absolute Anzahl der aus dem Intervall D ausgewählten Messwerte ist zwar größer als die Anzahl der Messwerte aus dem Intervall B; dennoch ist die Messwertdichte der selektierten Werte aus dem Intervall B höher.

Figur 5 zeigt, dass im Intervall B die Messwerte mit einem konstanten Raster selektiert werden, während im Intervall D die Messwerte mit einem Raster ausgewählt werden, das auf einer Wahrscheinlichkeitsfunktion beruht. Die zeitlichen Abstände der ausgewählten Messwerte sind hier nicht äquidistant.

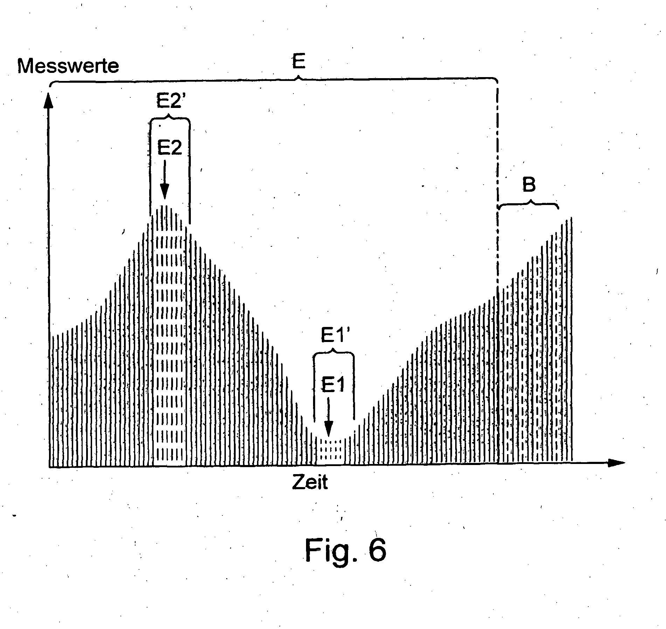

In Figur 6 ist der Fall gezeigt, dass bei einer Einteilung der Gesamtheit der Messwerte in zwei Intervalle B,E im zweiten Intervall E zwei Messwerte E1 und E2 ausgewählt wurden, die als auffällig charakterisiert werden, da sie außerhalb eines Toleranzbandes liegen. Obwohl die Selektion der Messwerte im Intervall E mit einem variablen Raster erfolgt, wird nach Detektion der Auffälligkeit der Messwerte E1 und E2 die Auswahlstrategie derart verändert, dass um die auffälligen Messwerte E1, E2 je ein Intervall E1' und E2' gebildet wird, in dem die Messwerte mit einem konstanten Raster selektiert werden. Im vorliegenden Fall werden dabei alle Messwerte innerhalb des Intervalls E1' und E2' ausgewählt. Somit kann das Analysesystem sehr flexibel auf auffällige Messwerte reagieren. Der hohe Informationsgehalt, der den auffälligen Werten und den zu ihnen benachbarten Werten zugrunde liegt, wird bei der Selektion der ersten Teilmenge berücksichtigt. Die selektierten Messwerte der ersten Teilmenge bilden damit die Gesamtheit der Messwerte hinreichend genau ab, um aus der ersten Teilmenge der Messwerte bereits eine Aussage über alle Messwerte zu machen, beispielsweise um den Gesundheitszustand des Patienten oder den Systemzustand einer Insulinpumpe beurteilen zu können.

## Patentansprüche

1. Diabatescare-System zur Detektion eines für die Behandlung des Diabetes Mellitus relevanten Analyten im menschlichen Körper mit einer mobilen Komponente (2), wie einem Analysegerät, einem Blutzuckermessgerät, einem Insulinpen oder einer Insulinpumpe, und einer Basisstation (3), wobei
die mobile Komponente (2) am Körper tragbar oder in den Körper implantierbar ist, und
einen Speicher (7) zum Speichern von Daten betreffend Dosiervolumina, Fördervolumina, Druckverläufe, Messwerte oder Analysedaten, und
eine Kommunikationseinheit (5) zur Übertragung von Daten an die Basisstation (3)
einschließt,
die Basisstation (3)
eine Kommunikationseinheit (8) um drahtlosen Empfangen von Daten von der mobilen Komponente und
eine Speichereinheit (9) zum Abspeichern von übertragenen Daten und
optional eine Ausgabeeinheit (10) zur Anzeige von von der mobilen Komponente übertragenen Daten
aufweist, und
eine Datenübertragung derartig selektiv durchgeführt wird, dass innerhalb einer ersten Zeitspanne, in der eine drahtlose Kommunikationsverbindung besteht, eine erste Teilmenge der Daten von der mobilen Komponente (2) an die Basisstation (3) übertragen wird,
die erste Teilmenge der Daten mittels eines prozessorgesieuerten Selektionsalgorithmus, der von einem Prozessor (6) der mobilen Komponente (2) gesteuert wird, derartig selektiert wird, dass die in der ersten Zeitspanne übertragene Teilmenge der Daten für die Gesamtheit der in der mobilen Komponente (2) abgespeicherten Daten repräsentativ ist und damit die Gesamtheit der Daten abbildet,
wobei eine Auswahl weiterer Daten in Abhängigkeit von einem Datanwert bereits selektierter Daten getroffen wird und die weiteren Daten innerhalb eines Zeitintervalls um Daten mit einem auffälligen Datenwert, der außerhalb eines vorgegebenen, vorzugsweise veränderbaren, Toleranzbandes liegt, selektiert werden und die weiteren Daten bei der Selektion der ersten Teilmenge berücksichtigt werden.

2. Diabetescare-System nach Anspruch 1, **dadurch gekennzeichnet, dass** die erste Teilmenge der Daten vor Beginn der Datenübertragung selektiert wird.

3. Diabetescare-System nach Anspruch 1 oder 2, **dadurch gekennzeichnet, dass** mindestens ein Teil der ersten Teilmenge der Daten in einer Reihenfolge, die umgekehrt zu der Reihenfolge ihrer Erzeugung ist, an die Basisstation (3) übertragen wird.

4. Diabetescare-System nach einem der vorhergehenden Ansprüche, **dadurch gekennzeichnet, dass** die erste Teilmenge der Daten in einem festen zeitlichen Raster so selektiert wird, dass der zeitliche Abstand zwischen benachbarten selektierten Daten äquidistant ist.

5. Diabetescare-System nach einem der Ansprüche 1 bis 3, **dadurch gekennzeichnet, dass** die erste Teilmenge der Daten mittels eines Selektionsalgorithmus, in dem eine Wahrscheinlichkeitsfunktion implementiert ist, so selektiert wird, dass der zeitliche Abstand zwischen benachbarten selektierten Daten verschieden ist.

6. Diabetescare-System nach einem der vorhergehenden Ansprüche, **dadurch gekennzeichnet, dass** die Gesamtheit der in dem Speicher (7) gespeicherten Daten in mehrere Intervalle unterteilt wird und die erste Teilmenge derart aus Daten der Intervalle zusammengesetzt wird, dass aus den Intervallen die Daten mit unterschiedlicher Dichte dergestalt selektiert werden, dass die intervalle unterschiedlich gewichtet werden.

7. Diabetescare-System nach dem vorhergehenden Anspruch, **dadurch gekennzeichnet, dass** die Gesamtheit der gespeicherten Daten in mindestens zwei intervalle unterteilt wird, wobei die Daten eines ersten Intervalls jünger sind als die Daten eines zweiten Intervalls sind, und die erste Teilmenge derart selektiert wird, dass die Dichte der aus dem ersten Intervall selektierten Daten größer ist als die Dichte der aus dem zweiten Intervall selektierten Daten.

8. Diabetescare-System nach einem der vorhergehenden Ansprüche, **dadurch gekennzeichnet, dass** die voraussichtliche Anzahl der Daten der ersten Teilmenge und/oder die voraussichtliche Dauer der ersten Zeitspanne für eine Datenübertragung aus bereits durchgeführten Datenübertragungen zwischen der mobilen Komponente (2) und der Basisstation (3) ermittelt wird.

9. Diabetescare-System nach einem der vorhergehenden Ansprüche, **dadurch gekennzeichnet, dass** an der Basisstation (3) eine Meldeeinrichtung (11) vorgesehen sind, um eine optische und/oder akustische Warnmeldung auszugeben, wenn die Anzahl der übertragenen Daten eine unter Grenze unterschreitet.

10. Diabetescare-System nach einem der vorhergehenden Ansprüche, **dadurch gekennzeichnet, dass** die Anzahl der Daten der ersten Teilmenge, die von der mobilen Komponente (2) an die Basisstation (3) übertragen worden ist, in der mobilen Komponente (2) und/oder in der Basisstation (3) registriert wird.

11. Diabetescare-System nach einem der vorhergehenden Ansprüche, **dadurch gekennzeichnet, dass** übertragene Daten aus dem Speicher (7) der mobilen Komponente (2) gelöscht werden.

12. Diabetescare-System nach einem der vorhergehenden Ansprüche, **dadurch gekennzeichnet, dass** es als Analysesystem zur Detektion eines Analyten in einem menschlichen Körper ausgebildet ist und eine mobile Komponente aufweist, welches eine Messeinheit zur Detektion das Analyten und Erzeugung von für die Analyse charakteristischen Daten einschließt.

13. Verfahren zur selektiven Datenübertragung innerhalb eines Diebetescare-Systems zur Detektion eines für die Behandlung des Diabetes Mallitus relevanten Analyten im menschlichen Körper mit einer mobilen Komponente (2), wie einem Analysengerät, einem Blutzuckermessgerät, einem Insulinpen oder einer Insulinpumpe, und einer Basisstation (3), wobei
die mobile Komponente (2) am Körper tragbar oder in den Körper implantierbar ist, und
einen Speicher (7) zum Speichern von Daten betreffend Dosiervolumina, Fördervolumina, Druckverläufe, Messwerte oder Analysedaten, und
eine Kommunikationseinheit (5) zur Übertragung von Daten an die Basisstation (3)
einschließt,
die Basisstation (3)
eine Kommunikationseinheit (8) zum drahtlosen Empfangen von Daten von der mobilen Komponente (2) und
eine Speichereinheit (9) zum Abspeichern von übertragenen Daten und
optional eine Ausgabeeinheit (10) zur Anzeige von von der mobilen Komponente (2) übertragenen Daten
aufweist, und
wobei selektiv eine Datenübertragung mit den folgenden Schritten durchgeführt wird:
Selektieren einer ersten Teilmenge der Daten mittels eines prozessorgesteuerten Selektionsalgorithmus in der mobilen Komponente (2);
Übertragen der selektierten ersten Teilmenge der Daten von der mobilen Komponente (2) an die Basisstation (3) innerhalb einer ersten Zeitspanne, in der eine drahtlose Kommunikationsverbindung besteht,
wobei die erste Teilmenge der Daten mittels eines prozessorgesteuerten Selektionsalgorithmus, der von einem Prozessor (6) der mobilen Komponente (2) gesteuert wird, derartig selektiert wird, dass die in der ersten Zeitspanne übertragene Teilmenge der Daten für die Gesamtheit der in der mobilen Komponente (2) abgespeicherten Daten repräsentativ ist und damit die Gesamtheit der Daten abbildet,
wobei eine Auswahl weiterer Daten in Abhängigkeit von einem Datenwert bereits ausgewählter Daten getroffen wird und die weiteren Daten innerhalb eines Zeitintervalls um Daten mit einem auffälligen Datenwert, der außerhalb eines vorgegebenen, vorzugsweise veränderbaren, Toleranzbandes liegt, selektiert werden und die weiteren Daten bei der Selektion der ersten Teilmenge berücksichtigt werden.

## Claims

1. Diabetes care system for the detection of an analyte in the human body, the analyte being relevant for the treatment of diabetes mellitus having a mobile component (2), such as an analysis device, a blood sugar measuring device, an insulin pen or an insulin pump, and a base station (3), wherein
the mobile component (2) being wearable on the body or implantable in the body, and including
a memory (7) for storing data regarding dosing volume, delivery volume, pressure curves, measuring data or analysis data, and
a communication unit (5) for transmitting data to the base station (3),
the base station (3) having
a communication unit (8) for wirelessly receiving data from the mobile component and
a memory unit (9) for storing transmitted data and
optionally an output unit (10) for displaying data transmitted from the mobile component, and
a data transmission is selectively performed in such a manner that within a first time interval, in which a wireless communication link exists, a first partial set of the data is transmitted from the mobile component (2) to the base station (3),
the first partial set is selected using a processor-controlled selection algorithm, which is controlled by a processor (6) of the mobile component (2), in such a manner that the data transmitted in the first time interval are representative of the entirety of the data stored in the mobile component (2) and so represents the entirety of data,
wherein the selection of further data can be made depending on a data volume of already selected data and the further data are selected in a time interval around data having an outlier data value, which lies outside a predefined, preferably changeable, tolerance band, and further data being considered during the selection of the first partial set.

2. Diabetes care system according to Claim 1, **characterized in that** the first partial set of the data is selected before beginning the data transmission.

3. Diabetes care system according to any one of Claims 1 or 2, **characterized in that** at least a part of the first partial set of the data is transmitted to the base station (3) in a sequence which is reversed from the sequence of its generation.

4. Diabetes care system according to any one of the preceding claims, **characterized in that** the first partial set of the data is selected in a fixed time raster in such a manner that the time interval between neighboring selected items of data is equidistant.

5. Diabetes care system according to any one of Claims 1 to 3, **characterized in that** the first partial set of the data is selected using a selection algorithm, in which a probability function is implemented, in such a manner that the time interval between neighboring selected items of data differs.

6. Diabetes care system according to any one of the preceding claims, **characterized in that** the entirety of the data stored in the memory (7) is divided into multiple intervals and the first partial set is composed of data of the intervals in such a manner that the data is selected from the intervals at varying density so that the intervals are differently weighted.

7. Diabetes care system according to the preceding Claim, **characterized in that** the entirety of the stored data is divided into data generated during at least two intervals, the data of a first interval being more recent than the data of the second interval, and the first partial set is selected in such a manner that the density of the data selected from the first interval is greater than the density of the data selected from the second interval.

8. Diabetes care system according to any one of the preceding claims, **characterized in that** an expected number of data of the first partial set and/or an expected duration of the first time interval for a data transmission is determined on the basis of already performed data transmissions between the mobile component (2) and the base station (3).

9. Diabetes care system according to any one of the preceding claims, **characterized in that** an alarm unit (11) is provided as part of the base station (3) to output an optical and/or acoustic warning message if the number of transmitted data falls below a lower limit.

10. Diabetes care system according to any one of the preceding claims, **characterized in that** the number of data of the first partial set which have been transmitted by the mobile component (2) to the base station (3) is registered in the mobile component (2) and/or in the base station (3).

11. Diabetes care system according to any one of the preceding claims, **characterized in that** transmitted data are erased from the memory (7) of the mobile component (2).

12. Diabetes care system according to any one of the preceding claims, **characterized in that** it is implemented as an analysis system for detecting an analyte in a human body and has a mobile component which includes a measuring unit for detecting the analyte and generating characteristic data for the analysis.

13. Method for selective data transmission within a diabetes care system for the detection of an analyte in the human body, the analyte being relevant for the treatment of diabetes mellitus having a mobile component (2), such as an analysis device, a blood sugar measuring device, an insulin pen or an insulin pump, and a base station (3), wherein
the mobile component (2) being wearable on the body or implantable in the body, and including
a memory (7) for storing data regarding dosing volume, delivery volume, pressure curves, measuring data or analysis data, and
a communication unit (5) for transmitting data to the base station (3),
the base station (3) having
a communication unit (8) for wirelessly receiving data from the mobile component (2) and
a memory unit (9) for storing transmitted data and
optionally an output unit (10) for displaying data transmitted from the mobile component (2),
wherein a data transmission is selectively performed using the following steps:
selecting a first partial set of the data using a processor-controlled selection algorithm in the mobile component (2);
transmitting the first partial set of the data from the mobile component (2) to the base station (3) within a first time interval, in which a wireless communication link exists,
wherein the first partial set is selected using a processor-controlled selection algorithm, which is controlled by a processor (6) of the mobile component (2), in such a manner that the data transmitted in the first time interval are representative of the entirety of the data stored in the mobile component (2) and so represents the entirety of data,
wherein the selection of further data can be made depending on a data volume of already selected data and the further data are selected in a time interval around data having an outlier data value,
which lies outside a predefined, preferably changeable, tolerance band, and further data being considered during the selection of the first partial set.

## Revendications

1. Système de surveillance du diabète pour une détection d'un analyte pertinent pour le traitement du diabète sucré dans un corps humain avec une composante mobile (2), comme un appareil d'analyse, un appareil de mesure de la glycémie, un stylo à insuline ou une pompe à insuline, et une station de base (3), dans lequel
la composante mobile (2) peut être portée sur le corps ou peut être implantée dans le corps, et comprend
une mémoire (7) pour une mémorisation de données concernant des volumes de dosage, des volumes de transport, des courbes de tension,
des valeurs de mesure ou des données d'analyse, et
une unité de communication (5) pour un transfert de données vers la station de base (3)
la station de base (3) présente
une unité de communication (8) pour une réception sans fil de données en provenance de la composante mobile et
une unité de mémorisation (9) pour une mise en mémoire de données transférées et
éventuellement une unité de sortie (10) pour un affichage de données transférées à partir de la composante mobile, et
un transfert de données est mis en oeuvre de manière sélective de sorte qu'une première quantité partielle des données en provenance de la composante mobile (2) est transférée vers la station de base (3) à l'intérieur d'un premier laps de temps dans lequel a lieu une liaison de communication sans fil, la première quantité partielle des données est sélectionnée au moyen d'un algorithme de sélection commandé par processeur, qui est commandé par un processeur (6) de la composante mobile (2), de sorte que la quantité partielle des données transférée dans le premier laps de temps est représentative de la totalité des données mémorisées dans la composante mobile (2) et représente ainsi la totalité des données,
dans lequel un choix d'autres données est effectué en fonction d'une valeur de données issue de données déjà sélectionnées et les autres données sont sélectionnées à l'intérieur d'un laps de temps pour des données présentant une valeur de données remarquable qui se situe à l'extérieur d'une bande de tolérance prédéfinie, de manière préférée modifiable, et les autres données sont prises en compte lors de la sélection de la première quantité partielle.

2. Système de surveillance du diabète selon la revendication 1, **caractérisé en ce que** la première quantité partielle des données est sélectionnée avant le début du transfert de données.

3. Système de surveillance du diabète selon la revendication 1 ou 2, **caractérisé en ce qu'**au moins une partie de la première quantité partielle des données est transférée vers la station de base (3) dans un ordre qui est inverse de l'ordre de sa production.

4. Système de surveillance du diabète selon l'une quelconque des revendications précédentes, **caractérisé en ce que** la première quantité partielle des données est sélectionnée dans une trame temporelle fixe de telle manière que l'écart temporel entre des données sélectionnées voisines est équidistant.

5. Système de surveillance du diabète selon l'une quelconque des revendications 1 à 3, **caractérisé en ce que** la première quantité partielle des données est sélectionnée au moyen d'un algorithme de sélection dans lequel est implémentée une fonction de probabilité, de sorte que l'écart temporel entre des données sélectionnées voisines est différent.

6. Système de surveillance du diabète selon l'une quelconque des revendications précédentes, **caractérisé en ce que** la totalité des données mémorisées dans la mémoire (7) est divisée en plusieurs laps et la première quantité partielle est agrégée à partir de données des laps de telle manière que, à partir des laps, les données présentant une densité différente sont sélectionnées de telle manière que les laps sont pondérés de manière différente.

7. Système de surveillance du diabète selon l'une quelconque des revendications précédentes, **caractérisé en ce que** la totalité des données mémorisées est divisée en au moins deux laps, dans lequel les données d'un premier laps sont plus récentes que les données d'un second laps, et la première quantité partielle est sélectionnée de telle manière que la densité des données sélectionnées à partir du premier laps est plus grande que la densité des données sélectionnées à partir du second laps.

8. Système de surveillance du diabète selon l'une quelconque des revendications précédentes, **caractérisé en ce que** le nombre probable des données de la première quantité partielle et/ou la durée probable du premier laps de temps pour un transfert de données est déterminée à partir de transferts de données déjà effectués entre la composante mobile (2) et la station de base (3).

9. Système de surveillance du diabète selon l'une quelconque des revendications précédentes, **caractérisé en ce qu'**un dispositif avertisseur (11) est prévu au niveau de la station de base (3), afin d'émettre un message d'avertissement optique et/ou acoustique lorsque le nombre des données transférées passe en dessous d'une limite inférieure.

10. Système de surveillance du diabète selon l'une quelconque des revendications précédentes, **caractérisé en ce que** le nombre des données de la première quantité partielle, qui a été transféré de la composante mobile (2) vers la station de base (3), est enregistré dans la composante mobile (2) et/ou dans la station de base (3).

11. Système de surveillance du diabète selon l'une quelconque des revendications précédentes, **caractérisé en ce que** des données transférées sont supprimées de la mémoire (7) de la composante mobile (2).

12. Système de surveillance du diabète selon l'une quelconque des revendications précédentes, **caractérisé en ce qu'**il est réalisé en tant que système d'analyse destiné à une détection d'un analyte dans un corps humain et présente une composante mobile qui comprend une unité de mesure pour une détection dudit analyte et pour une production de données caractéristiques de l'analyse.

13. Procédé de transfert sélectif de données à l'intérieur d'un système de surveillance du diabète pour une détection d'un analyte pertinent pour le traitement du diabète sucré dans un corps humain avec une composante mobile (2), comme un appareil d'analyse, un appareil de mesure de la glycémie, un stylo à insuline ou une pompe à insuline, et une station de base (3), dans lequel
la composante mobile (2) peut être portée sur le corps ou peut être implantée dans le corps, et comprend
une mémoire (7) pour une mémorisation de données concernant des volumes de dosage, des volumes de transport, des courbes de tension, des valeurs de mesure ou des données d'analyse, et
une unité de communication (5) pour un transfert de données vers la station de base (3),
la station de base (3) présente
une unité de communication (8) pour une réception sans fil de données en provenance de la composante mobile (2) et
une unité de mémorisation (9) pour une mise en mémoire de données transférées et
éventuellement une unité de sortie (10) pour un affichage de données transférées à partir de la composante mobile (2), et
dans lequel un transfert de données est mis en oeuvre de manière sélective grâce aux étapes suivantes consistant à :
sélectionner une première quantité partielle des données au moyen d'un algorithme de sélection commandé par processeur dans la composante mobile (2) ;
transférer la première quantité partielle sélectionnée des données à partir de la composante mobile (2) vers la station de base (3) à l'intérieur d'un premier laps de temps dans lequel a lieu une liaison de communication sans fil,
dans lequel la première quantité partielle des données est sélectionnée au moyen d'un algorithme de sélection commandé par processeur, qui est commandé par un processeur (6) de la composante mobile (2), de telle manière que la quantité partielle des données transférée dans le premier laps de temps est représentative de la totalité des données mémorisées dans la composante mobile (2) et représente ainsi la totalité des données,
dans lequel un choix d'autres données est effectué en fonction d'une valeur de données issue de données déjà choisies, et les autres données sont sélectionnées à l'intérieur d'un laps de temps pour des données présentant une valeur de données remarquable, qui se situe à l'extérieur d'une bande de tolérance prédéfinie, de manière préférée modifiable, et les autres données sont prises en compte lors de la sélection de la première quantité partielle.
